# FASCICULE DE BREVET EUROPEEN

(11) **EP 1 909 695 B1**
(45) Date de publication et mention de la délivrance du brevet: **03.10.2012**
(21) Numéro de dépôt: 06778590.7
(22) Date de dépôt: 12.06.2006
(51) Int. Cl.: A61F 2/06

(54) **DISPOSITIF DE TRAITEMENT D'UN VAISSEAU SANGUIN.**
VORRICHTUNG ZUR BEHANDLUNG EINES BLUTGEFÄSSES
DEVICE FOR TREATING A BLOOD VESSEL

(30) Priorité: 15.06.2005 FR 0506081
(43) Date de publication de la demande: 16.04.2008
(73) Titulaire: Laboratoires Perouse, 60173 Ivry Le Temple (FR)
(72) Inventeur: STYRC, Mikolaj, L-8190 Kopstal (LU); WEN, Ning, F-60500 Chantilly (FR); PEROUSE, Eric, F-75016 Paris (FR)
(74) Mandataire: Blot, Philippe Robert Emile
(86) Numéro de dépôt international: PCT/FR2006/001321
(87) Numéro de publication internationale: WO 2006/134258

(56) Documents cités:
- WO-A1-2005/079705
- FR-A- 2 688 688
- FR-A- 2 863 160
- FR-A- 2 865 926
- US-A1- 2003 004 560
- US-A1- 2004 193 252

## Description

La présente invention concerne un dispositif de traitement d'un vaisseau sanguin selon le préambule de la revendication 1.

Un tel dispositif s'applique au largage dans un vaisseau sanguin d'implants comme des endoprothèses tubulaires couramment désignées par le terme anglais « stent », ou d'autres types d'endoprothèses comme des endovalves. US 2004/0193252 décrit un dispositif du type précité.

Un dispositif est aussi décrit dans FR-A-2 863 160. Dans ce dispositif, une endoprothèse est montée coaxialement sur un tuteur creux. Cette endoprothèse est maintenue dans son état rétracté à l'aide de deux liens filiformes la ceinturant à ses extrémités.

Les liens filiformes sont engagés respectivement dans des ouvertures de retenue distale et proximale ménagées dans le tuteur.

Chaque lien filiforme comprend un tronçon de commande qui s'étend dans le tuteur jusqu'à une extrémité de commande respective accessible à l'utilisateur à travers un embranchement latéral respectif du tuteur.

Dans ce dispositif, chaque extrémité de l'endoprothèse peut être larguée indépendamment de l'autre extrémité.

A cet effet, le tronçon de commande d'un des liens filiformes est déplacé dans le tuteur vers l'extrémité distale pour relâcher l'extrémité correspondante de l'endoprothèse. Le lien filiformes est alors extrait du dispositif lorsque l'endoprothèse est placée de manière satisfaisante dans le vaisseau.

Pour la mise en place du dispositif dans le vaisseau sanguin du patient, un guide chirurgical est préalablement positionné dans le vaisseau sanguin. Puis, le tuteur portant l'endoprothèse est introduit dans le vaisseau par coulissement le long du guide. Lors de cette introduction, ou de la manoeuvre ultérieure des liens filiformes, ces liens et le guide peuvent s'entremêler, ce qui nuit à la fiabilité d'utilisation du dispositif.

Un but de l'invention est donc de proposer un dispositif de traitement d'un vaisseau sanguin qui peut être positionné de manière précise dans le vaisseau, et qui présente une fiabilité accrue.

A cet effet, l'invention a pour objet un dispositif selon la revendication 1.

Le dispositif selon l'invention peut comprendre une ou plusieurs des caractéristiques des revendications 2 à 12.

L'invention sera mieux comprise à la lecture de la description qui va suivre, donnée uniquement à titre d'exemple et faite en se référant aux dessins annexés, sur lesquels :
- la Figure 1 est une vue en élévation d'un premier dispositif de traitement selon l'invention ;
- la Figure 2 est une vue partielle et agrandie, en coupe suivant un plan longitudinal médian du dispositif de la Figure 1 ;
- la Figure 3 est une vue en coupe partielle à plus grande échelle suivant un plan transversal III-III d'un détail de la Figure 1 ;
- la Figure 4 est une vue d'un détail de la Figure 2 ;
- la Figure 5 est une vue analogue à la Figure 1, l'endoprothèse étant déployée de manière réversible ;
- la Figure 6 est une vue partielle en coupe suivant un plan longitudinal médian d'un deuxième dispositif selon l'invention;
- la Figure 7 est une vue analogue à la Figure 3, d'un troisième dispositif selon l'invention ; et
- la Figure 8 est une vue analogue à la Figure 4 du troisième dispositif selon l'invention.

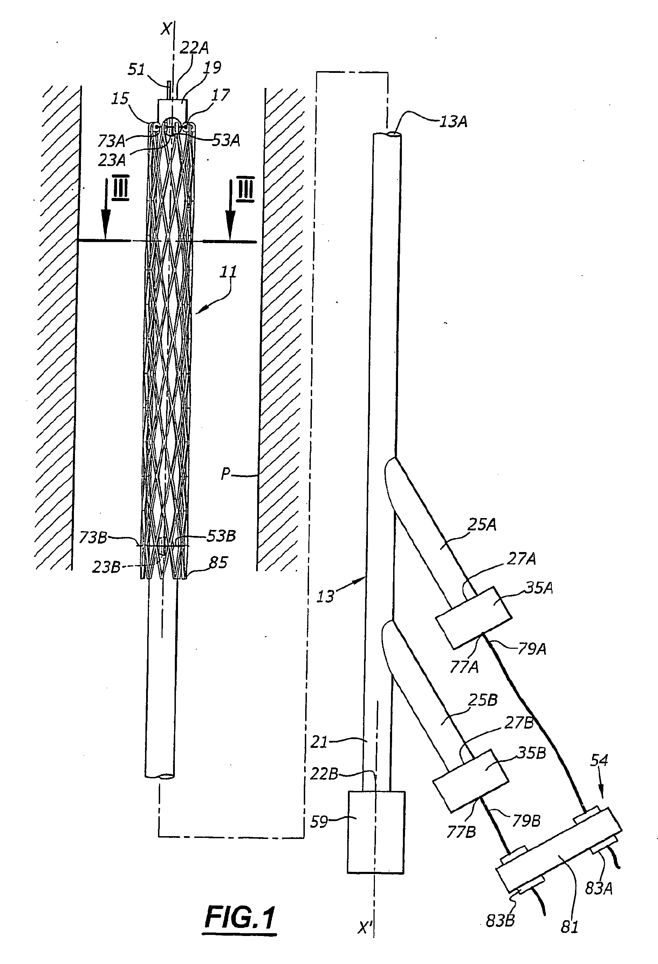

Le dispositif représenté sur les Figures 1 à 5 comporte une endoprothèse tubulaire 11 montée de manière coaxiale sur un tuteur unique 13 et reliée à ce tuteur 13 par des moyens de retenue libérables.

L'endoprothèse 11 comprend un treillis tubulaire d'acier inoxydable qui possède des propriétés de ressort. Ainsi, cette endoprothèse est auto-expansible.

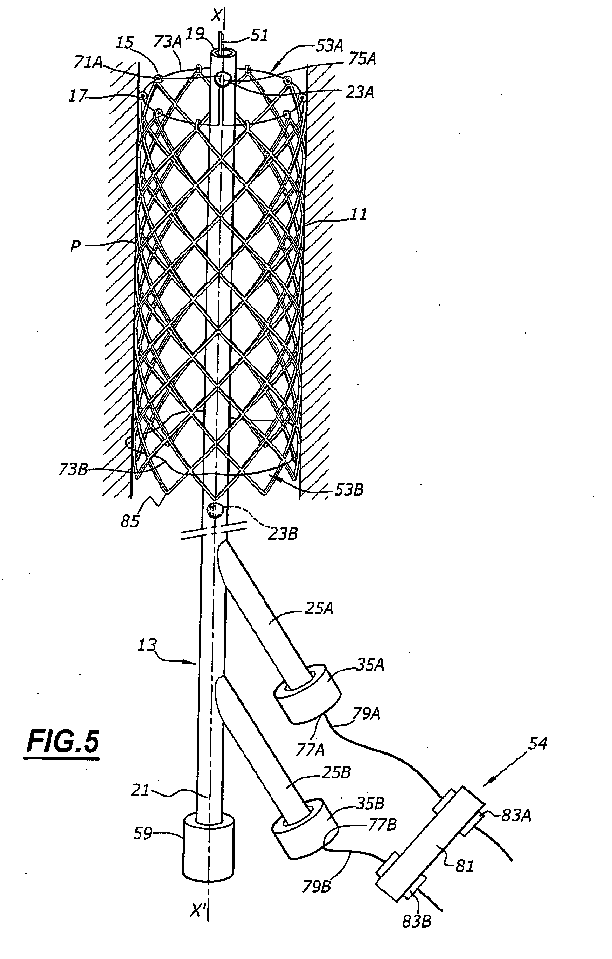

Comme connu en soi, l'endoprothèse 11 est susceptible de se déformer spontanément d'un état comprimé, dans lequel elle présente un petit diamètre (figure 1) à un état dilaté, dans lequel elle présente un diamètre supérieur (figure 5), cet état dilaté constituant son état de repos.

Le treillis présente à une extrémité distale 15 de l'endoprothèse, des fils repliés en boucles 17.

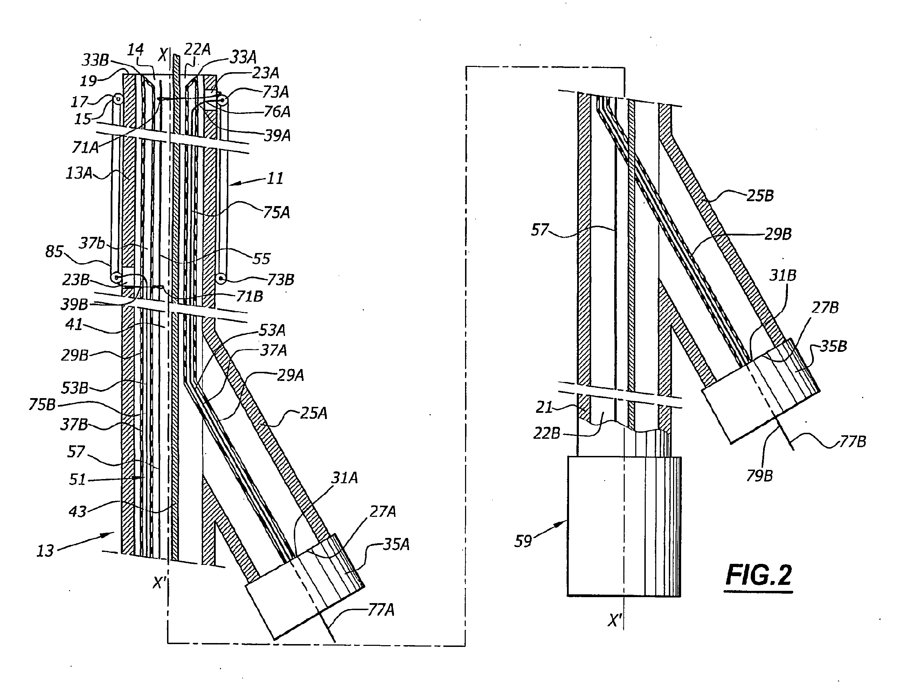

Dans l'exemple illustré sur les Figures 1 et 2, le tuteur 13 comprend une paroi tubulaire 13A métallique flexible creuse qui délimite un passage central 14.

Le tuteur 13 s'étend longitudinalement le long d'un axe longitudinal X-X' entre une extrémité distale 19 destinée à être implantée dans le vaisseau sanguin et une extrémité proximale 21 destinée à être accessible pour un chirurgien.

Le tuteur présente à ses extrémités distale et proximale 19, 21 des ouvertures de passage 22A, 22B respectives d'axe X-X' débouchant dans le passage central 14.

Des ouvertures de retenue distale et proximale 23A et 23B, décalées longitudinalement, sont ménagées latéralement dans le tuteur 13. Dans cet exemple, les ouvertures 23A et 23B sont ménagées sur des côtés opposés par rapport à un plan longitudinal médian du tuteur 13.

La distance qui sépare l'ouverture de retenue distale 23A de l'ouverture de retenue proximale 23B est sensiblement égale à la longueur de l'endoprothèse 11 dans son état rétracté, prise suivant une direction longitudinale.

Le tuteur 13 comprend par ailleurs des embranchements creux distal et proximal 25A, 25B au voisinage de son extrémité proximale 21. Ces embranchements 25A et 25B sont décalés longitudinalement le long du tuteur 13 et communiquent avec le passage 14 à l'intérieur du tuteur 13. Un passage de commande 27A, 27B est ménagé à une extrémité libre de chaque embranchement 25A, 25B.

Comme illustré par la Figure 2, le tuteur 13 comprend, pour chaque ouverture de retenue 23A, 23B, un conduit interne 29A, 29B d'isolation des moyens de retenue libérables raccordé à un embranchement 25A, 25B respectif.

Les conduits 29A, 29B sont réalisés à partir d'un matériau plastique souple présentant une tenue autonome. Ils sont formés par des tubes qui présentent une section cylindrique.

Chaque conduit d'isolation 29A, 29B s'étend longitudinalement dans le passage central 14 entre une extrémité proximale 31A, 31 B engagée dans le passage de commande 27A, 27B et une extrémité distale 33A, 33B située à l'extrémité distale 19 du tuteur dans le passage 14.

L'extrémité proximale 31A, 31B de chaque conduit d'isolation 29A, 29B est munie d'un bouchon 35A, 35B d'obturation du passage de commande 27 vissé sur l'embranchement 25A, 25B correspondant.

Chaque conduit d'isolation 29A, 29B est obturé à son extrémité distale 33A, 33B, qui forme un biseau divergeant vers l'extrémité distale du tuteur 13.

Les conduits 29A, 29B délimitent intérieurement des canaux 37A, 37B de réception des moyens de retenue libérables.

Chaque conduit 29A, 29B définit un passage distal 39A, 39B qui s'étend longitudinalement sensiblement en regard d'une ouverture de retenue 23A, 23B associée et qui débouche dans le canal 37A, 37B.

Chaque conduit d'isolation 29A, 29B est ainsi fixé au tuteur 13 par son extrémité proximale 31A, 31 B mais reste libre à son extrémité distale 33A, 33B.

En variante, chaque conduit 29A, 29B est également fixé au tuteur 13 à son extrémité distale 33A, 33B par un point de colle.

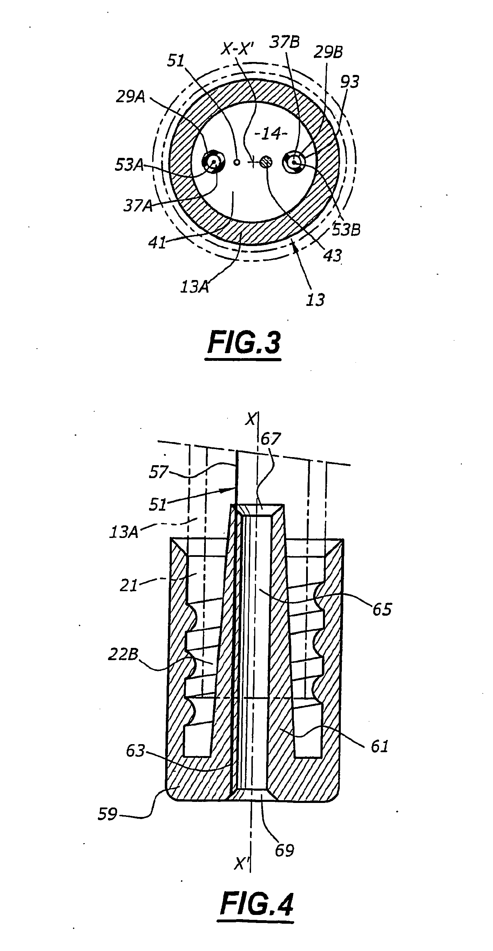

Comme illustré par la Figure 3, chaque canal 37A, 37B présente une section transversale cylindrique qui s'étend sur une partie d'une circonférence autour de l'axe X-X' du passage central 16.

Dans l'exemple représenté, les conduits 29A, 29B sont situés sur une même circonférence autour de cet axe.

De même, chaque canal 37A, 37B s'étend radialement en section transversale sur une partie de la distance qui sépare l'axe X-X' de la paroi tubulaire 13A.

Comme illustré par la Figure 3, l'espace situé dans le passage 14 entre les conduits d'isolation 29A, 29B et la paroi 13A forme un conduit 41 de circulation et de guidage d'un guide chirurgical 43. Les conduits d'isolation 29A et 29B sont répartis à la périphérie du conduit de circulation 41.

Les moyens de retenue libérables de l'endoprothèse 11 comprennent une tige de retenue 51, des fils de retenue distal et proximal 53A et 53B, et un palonnier 54 de commande des fils de retenue.

La tige de retenue 51 est disposée dans le passage central 14. La longueur de la tige 51 est supérieure ou égale à la distance entre l'ouverture de retenue distale 23A et l'extrémité proximale 21 du tuteur 13. Comme illustré sur la Figure 2, elle comprend une partie active 55 située au voisinage de l'extrémité distale 19 et une partie d'actionnement 57 qui s'étend jusqu'à l'extrémité proximale 21 du tuteur 13.

La tige 51 est mobile en translation dans le tuteur 13, entre une position de retenue dans laquelle la partie active 55 de la tige est en regard des deux ouvertures de retenue 23A et 23B, une position intermédiaire dans laquelle la partie active 55 est en regard de l'ouverture de retenue proximale 23B et à l'écart de l'ouverture de retenue distale 23A et une position de libération dans laquelle la partie active 55 est à l'écart des deux ouvertures de retenue 23A et 23B.

La tige 51 est fixée à l'extrémité proximale 21 du tuteur par un bouchon amovible 59 qui obture l'ouverture de passage proximale 22B.

Comme illustré par la Figure 4, le bouchon 59 présente un manchon central 61 d'axe X-X'. La base proximale de la tige 51 est noyée dans une paroi du manchon 61, de sorte que la tige 51 est décalée axialement par rapport à l'axe X-X'.

Le manchon 61 délimite un canal central 65 d'axe X-X' qui présente une entrée distale 67 dans le passage central 14 et une entrée proximale 69 à l'extérieur du tuteur 13. L'entrée distale 67 est délimitée par une surface du manchon 61 divergente distalement et l'entrée proximale 69 est délimitée par une surface divergente vers l'extérieur du tuteur 13.

Dans l'exemple représenté sur les Figures 1 à 5, chaque fil de retenue 53A, 53B comprend un brin unique, qui comporte un passant d'extrémité 71A, 71 B, une boucle de serrage 73A, 73B, et un tronçon de commande 75A, 75B.

En variante (non représentée), chaque fil de retenue 53A, 53B comprend deux brins parallèles reliés entre eux au moins par une partie d'extrémité. Le passant est formé directement par cette partie d'extrémité, engagée autour de la tige 51.

Le passant d'extrémité 71A, 71B est formé à une extrémité distale du brin. Il est formé d'une boucle fermée de petit diamètre. La partie active 55 de la tige 51 est engagée dans le passant 71A, 71 B, lorsque la tige 51 est dans sa position de retenue.

Le passant 71A, 71 B est par ailleurs déformable de sorte que sa largeur, lorsqu'il est déformé est sensiblement égale à deux fois la largeur du brin. Cette largeur est inférieure au diamètre interne des boucles 17.

Le passant 71A, 71 B est relié à la boucle de serrage 73A, 73B par un tronçon 76A engagé dans l'ouverture de retenue correspondante 23A, 23B.

Dans l'exemple illustré sur la Figure 1, la boucle de serrage 73A, 73B est formée par un tronçon du brin, engagé de manière coulissante dans les boucles d'extrémité 17 du treillis de l'endoprothèse 11, suivant une circonférence de cette endoprothèse 11, autour d'un axe longitudinal.

Chaque boucle de serrage 73A, 73B fixe l'endoprothèse 11 au tuteur 13.

Par ailleurs, la longueur active de la boucle de serrage 73A, 73B est variable, de sorte qu'elle contrôle le déploiement de l'endoprothèse 11 par rapport au tuteur 13, comme on va le décrire plus bas.

Comme représenté sur la Figure 2, chaque tronçon de commande 75A, 75B s'étend dans un conduit d'isolation 29A, 29B entre son passage distal 39A, 39B et le passage de commande 27A, 27B de l'embranchement de commande 25A, 25B correspondant.

Chaque conduit d'isolation 29A, 29B reçoit donc un tronçon de commande 75A, 75B unique qui est isolé du conduit 41. Par suite, le conduit 41 de circulation du guide 43 dans le passage central 14 est dépourvu de fil de retenue 53A, 53B entre l'extrémité proximale 21 du tuteur et l'ouverture de retenue proximale 23A, ce qui permet le passage du guide 43 sans interaction mécanique avec les fils 53A, 53B.

Une extrémité de commande 77A, 77B du tronçon de commande 75A, 75B est engagée à travers le passage de commande 27A, 27B. Ainsi, une partie 79A, 79B de ce tronçon fait saillie hors de l'embranchement 25A, 25B à travers le bouchon 35A, 35B. La longueur de cette partie en saillie 79A, 79B est variable et commande la longueur de la boucle de serrage 73A, 73B.

Ainsi, une augmentation de la longueur de la partie en saillie 79A, 79B provoque le déplacement du tronçon de commande 75A, 75B par rapport au tuteur 13, vers l'extrémité proximale 21 de ce tuteur, ainsi qu'une diminution correspondante de la longueur active de la boucle de serrage 73A, 73B, et par suite, le serrage de l'endoprothèse 11 contre le tuteur 13, au niveau de la boucle de serrage 73A, 73B.

Lorsque l'endoprothèse 11 est dans son état rétracté contre le tuteur 13, le tronçon de commande 75A, 75B est dans une position sous tension.

Inversement, une diminution de la longueur de la partie en saillie 79A, 79B provoque le déplacement du tronçon de commande 75A, 75B par rapport au tuteur 13 vers l'extrémité distale 19 du tuteur, ainsi qu'une augmentation de la longueur active de la boucle de serrage 73A, 73B et par suite, le déploiement de l'endoprothèse 11 à l'écart du tuteur 13, au niveau de la boucle de serrage 73A, 73B.

Lorsque l'endoprothèse 11 est dans son état dilaté, le tronçon de commande 75A, 75B est dans une position détendue.

Chaque bouchon 35A, 35B comprend une lumière centrale de diamètre ajustable dans laquelle est engagé la partie en saillie 79A, 79B.

En réglant le diamètre de la lumière centrale du bouchon 35A, 35B, on immobilise de manière sélective la partie en saillie 79A, 79B du tronçon de commande 75A, 75B par rapport au tuteur 13 et on fixe la longueur de la partie en saillie 79A, 79B et par suite, la longueur active de la boucle de serrage 73A, 73B.

Le palonnier de commande 54 comprend une poignée 81 et des moyens 83A, 83B de fixation libérables des extrémités des tronçons de commande 75A, 75B situés de part et d'autre de la poignée.

La poignée 81 est située hors du passage 14 à l'écart du tuteur 13.

Les extrémités libres des tronçons de commande 75A, 75B, au niveau des parties en saillie 79A, 79B sont fixées respectivement de part et d'autre de la poignée 81 du palonnier 54.

Le palonnier 54 permet l'actionnement simultané des deux fils de commande 53A, 53B à l'aide d'une seule main, comme on le verra plus bas.

On décrira maintenant comme exemple le fonctionnement du premier dispositif du traitement selon l'invention.

Dans un premier temps, le dispositif est conservé dans un emballage (non représenté), avec l'endoprothèse 11 dans un état déployé analogue à celui représenté sur la figure 5.

Dans cette configuration, la tige de commande 51 est dans sa position de retenue. Les fils de retenue distal et proximal 53A et 53B sont engagés dans la tige 51 et dans le treillis de l'endoprothèse 11.

Ce conditionnement conserve les propriétés mécaniques de l'endoprothèse 11, particulièrement lorsque le treillis tubulaire de celle-ci est noyé dans un film extensible et étanche, tel qu'un élastomère.

Dans un deuxième temps, le chirurgien extrait le dispositif de son emballage. Il implante le guide chirurgical 43 circulant dans le vaisseau sanguin ou la veine depuis le point d'introduction extérieur jusqu'à la zone de la veine ou de l'artère dans laquelle doit être implantée l'endoprothèse tubulaire.

Dans un troisième temps, en vue de l'implantation de l'endoprothèse 11 dans le vaisseau sanguin ou la veine, le chirurgien actionne simultanément les fils de retenue 53A et 53B en tirant la poignée 81 à l'écart du tuteur 13 afin d'augmenter la longueur de la partie en saillie 79A, 79B du tronçon de commande 75A, 75B. Il déplace ainsi simultanément les tronçons de commande 75A, 75B vers l'extrémité proximale 21 du tuteur 13. La longueur active de chaque boucle de serrage 73A, 73B diminue de sorte que l'endoprothèse 11 est rétractée contre le tuteur 13 et fixée solidement par rapport au tuteur 13.

Dans certains cas, et pour maintenir un encombrement radial minimal, un fourreau (non représenté) est disposé autour de l'endoprothèse 11, avant cette introduction et retiré une fois l'introduction effectuée.

L'endoprothèse 11 est alors dans l'état rétracté illustré sur la figure 1 dans lequel le treillis est sensiblement en appui contre le tuteur 13. Elle est ainsi introduite jusqu'à son lieu d'implantation par déplacement le long du guide chirurgical 43.

A cet effet, le guide 43 est introduit dans le passage central 14 par l'ouverture de passage distale 22A. L'introduction du guide 43 à l'intérieur du passage 14 est facilitée par la forme en biseau des extrémités distales des conduits 29A, 29B. Par ailleurs, les tronçons de commande 75A, 75B des fils 53A, 53B étant disposés dans les conduits 29A, 29B, le guide 43 est libre de circuler dans le conduit 41, sans rencontrer de fils 53A, 53B.

Lorsque l'extrémité du guide 43 atteint l'extrémité proximale 21 du tuteur, le guide 43 pénètre dans le canal central 65 du bouchon 59. La forme divergente du manchon 61 au niveau de l'entrée 67 permet également un guidage aisé du guide 43. Le tuteur portant l'endoprothèse est ensuite déplacé le long du guide 43.

Une fois l'endoprothèse 11 introduite, le chirurgien procède à son déploiement.

En fonction de la conformation du vaisseau à traiter, il peut choisir de déployer en premier l'une ou l'autre des extrémités de l'endoprothèse 11.

On décrira comme exemple le déploiement de l'extrémité distale 15.

Tout d'abord, le chirurgien actionne diminue progressivement la longueur de la partie en saillie 79A du tronçon de commande 75A en le libérant du bouchon 35A. A l'aide du palonnier 54, il déplace le tronçon de commande 75A vers l'extrémité distale 19 du tuteur 13. Par suite, la longueur active de la boucle de serrage 73A augmente.

Le treillis de l'endoprothèse 11 se déforme alors spontanément de l'état comprimé représenté sur la figure 1 à l'état déployé représenté sur la Figure 4.

Durant cette déformation, les boucles d'extrémité 17 du treillis s'éloignent du tuteur 13 et se rapprochent des parois P du vaisseau à traiter, pour venir en appui sur ces parois P.

De manière analogue, le chirurgien effectue ensuite le déploiement de l'extrémité proximale 85 de l'endoprothèse, au moyen du fil de retenue proximal 53B (Figure 4).

Les tronçons de commande 75A, 75B étant isolés l'un de l'autre dans chaque conduit d'isolation 29A, 29B, leur déplacement s'effectue de manière fiable, et le risque de blocage des fils 53A, 53B est fortement diminué.

Lorsque le chirurgien est satisfait du positionnement de l'extrémité distale 15 de l'endoprothèse 11, il déplace la tige de retenue 51 depuis sa position de retenue jusqu'à la position intermédiaire. Lors de ce déplacement, le passant 71A du fil de retenue distal 53A est libéré de la tige 51.

Le chirurgien tire ensuite sur l'extrémité de commande 77A à l'aide du palonnier 54 pour amener l'extrémité distale du fil de retenue distal 23A jusqu'au passage de commande 27, successivement au travers des boucles 17 du treillis de l'endoprothèse 11, l'intérieur du tuteur 13, et l'embranchement de commande 25A.

En variante, les tronçons de commande 75A, 75B des fils de retenue 53A, 53B sont libérés simultanément de leurs bouchons 35A, 35B. Le chirurgien manoeuvre alors simultanément les deux tronçons de commande 75A, 75B à l'aide de la poignée 81 du palonnier. A cet effet, il peut déployer simultanément les deux extrémités 15, 85 de l'endoprothèse 11 en déplaçant les deux extrémités 83A, 83B de la poignée 81 vers les bouchons 35A, 35B. Il peut également déployer l'une des extrémités 15, 85 de l'endoprothèse en maintenant l'autre extrémité de l'endoprothèse rétractée contre le tuteur 13 en déplaçant une seule des extrémités 83A, 83B de la poignée 81 vers le bouchon 35A, 35B associé.

Le palonnier 54 facilite donc la manoeuvre du dispositif et permet de sélectionner d'une seule main le déploiement ou la rétractation de l'une et/ou de l'autre des extrémités de l'endoprothèse 11.

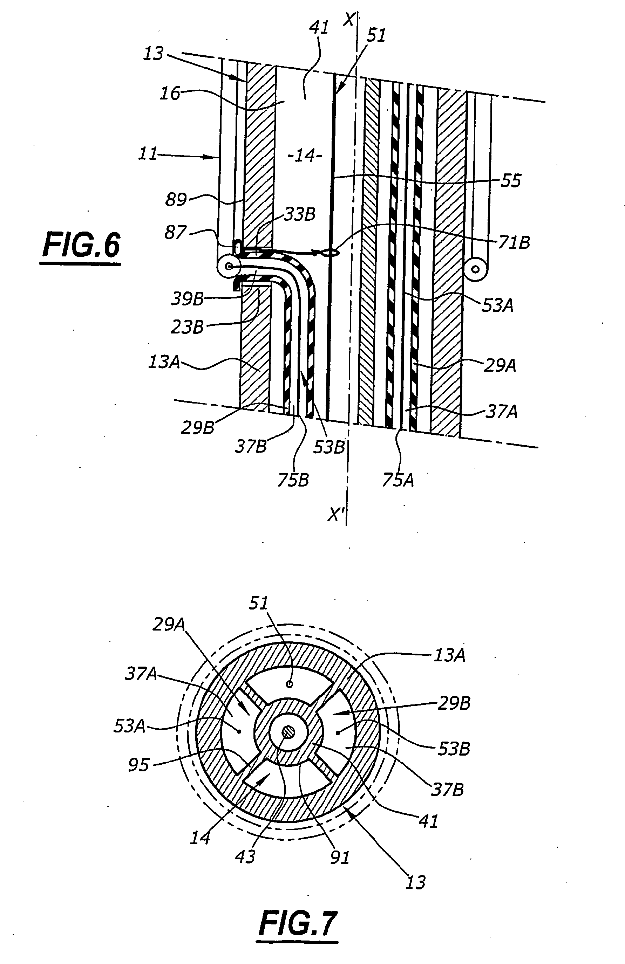

Dans le deuxième dispositif selon l'invention, représenté partiellement sur la Figure 6, le passage distal 39B est situé à l'extrémité distale 33B du conduit d'isolation 29B.

Le conduit d'isolation 29B présente par ailleurs autour du passage 39B à son extrémité distale 33B une collerette 87 d'application sur la paroi 13A du tuteur.

Le conduit 29B est coudé au voisinage de son extrémité distale 33B, laquelle est engagée à travers l'ouverture de retenue 23B. La collerette 85 s'appuie sur une surface extérieure 89 du tuteur 13 autour de l'ouverture 23B et fixe le conduit 29B au tuteur 13.

Dans le troisième dispositif selon l'invention, représenté sur la Figure 7, le tuteur 13 comprend un conduit central 91 de circulation du guide formé par une partie tubulaire d'axe X-X' disposée dans le passage central 14 et s'étendant jusqu'à l'extrémité proximale 21 du tuteur.

Le conduit central 91 est raccordé à la paroi 13A du tuteur 13 par des parois axiales planes 95.

Les parois axiales 95 présentent une section transversale s'étendant radialement à l'écart de l'axe X-X'. Elles délimitent entre le conduit central 91 et la paroi 13A une pluralité de conduits d'isolation 29A, 29B qui reçoivent les fils de retenue 53A, 53B.

Le conduit 91, la paroi 13A et les parois 95 sont venus de matière, le tuteur 13 pouvant être réalisé par exemple par extrusion.

Chaque canal 37A, 37B présente ainsi une section transversale en forme de secteur angulaire tronqué qui couvre une partie de la surface annulaire s'étendant entre le conduit 91 et la paroi 13A du tuteur autour de l'axe X-X'.

L'angle formé par deux parois axiales 95 adjacentes est par exemple compris entre 10 et 150 degrés.

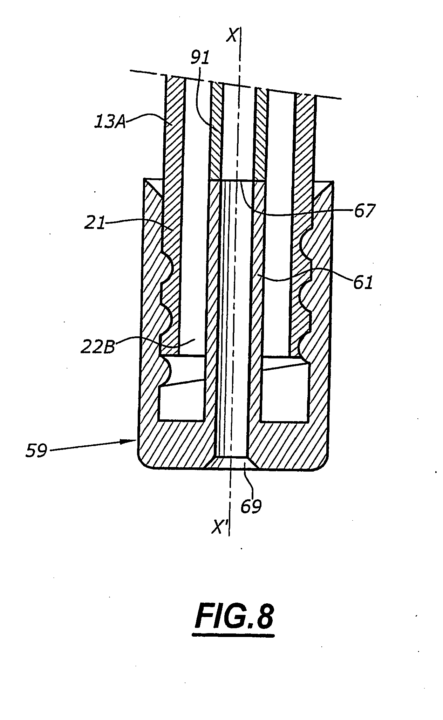

Comme illustré par la Figure 8, le bouchon 59 est formé de sorte que la surface interne du manchon 61 affleure intérieurement la surface interne du conduit 91, pour faciliter le passage du guide entre le conduit 91 et le bouchon 59.

Grâce à l'invention qui vient d'être décrite, il est possible de disposer d'un dispositif de traitement d'un vaisseau sanguin qui est facilement implantable sur un guide chirurgical 43. Ce dispositif est donc placé avec précision dans un vaisseau sanguin.

Les fils de retenue libérables 53A, 53B de l'endoprothèse 11 étant disposés dans des conduits d'isolation 29A, 29B, le risque d'interaction mécanique entre ces fils 53A, 53B et le guide 43 est fortement diminué.

Par ailleurs, la manoeuvre des tronçons de commande 75A, 75B de ces fils est rendue extrêmement fiable grâce à leur disposition dans les conduits internes d'isolation 29A, 29B.

L'utilisation d'un palonnier 54 reliant les extrémités libres des tronçons de commande 75A, 75B des fils de retenue 53A, 53B de l'endoprothèse simplifie considérablement le fonctionnement du dispositif et facilite son utilisation à l'aide d'une seule main.

On notera que le palonnier 54 s'applique à d'autres types de dispositifs de déploiement d'implants médicaux comprenant au moins deux fils de commande, notamment ceux dépourvus de conduits d'isolation 29 comme décrit dans la demande FR 2 863 160.

La présence de surfaces divergentes aux extrémités 19, 21 du tuteur 13 facilite l'insertion du guide 43 dans le passage central 14, soit de l'extrémité distale vers l'extrémité proximale du tuteur 13, soit dans le sens inverse.

## Revendications

1. Dispositif de traitement d'un vaisseau sanguin, du type comprenant:
- au moins un implant (11), déployable entre un état contracté et un état dilaté ;
- un tuteur creux (13), qui délimite intérieurement un passage central (14) entre une extrémité proximale (21) et une extrémité distale (19), le tuteur creux (13) définissant, au voisinage de son extrémité distale (19), au moins une ouverture de retenue (23A ; 23B) de l'implant;
- au moins un lien filiforme (53A, 53B) de fixation libérable de l'implant (11) au tuteur (13), le ou chaque lien filiforme (53A; 53B) comprenant un tronçon de traction (75A ; 76B) engagé dans l'ouverture de retenue (23A ; 23B) et s'étendant dans le passage central (14) au moins jusqu'à une extrémité de commande (77A, 77B) actionnable depuis l'extrémité proximale (21) du tuteur (13), le dispositif comprenant pour le ou chaque lien filiforme (53A ; 53B), un conduit interne d'isolation (29A ; 29B) ménagé dans le passage central (14) et fixe axialement par rapport au tuteur (13), le ou chaque conduit d'isolation (29A; 29B) recevant un seul lien (53A ; 53B) et délimitant, dans le passage central (14), un conduit de guidage (41) dépourvu de lien (53A ; 53B) au moins entre l'extrémité proximale (29) du tuteur et une ouverture de retenue (23A ; 23B), **caracterisé en ce que** le ou chaque conduit d'isolation est formé par un tube qui présente une section cylindrique.

2. Dispositif selon la revendication 1, **caractérisé en ce que** le ou chaque conduit d'isolation (29A, 29B) délimite un canal (37A ; 37B) de réception du lien s'étendant en section transversale sur une partie d'une circonférence autour d'un axe longitudinal (X-X') du passage central (14).

3. Dispositif selon l'une des revendications 1 ou 2, **caractérisé en ce que** le ou chaque conduit d'isolation (29A ; 29B) présente un passage distal (39A ; 39B) s'étendant sensiblement en regard d'une ouverture de retenue (23A, 23B) associée, le ou chaque lien (53A ; 53B) étant engagé dans le passage distal (39A ; 39B).

4. Dispositif selon l'une quelconque des revendications précédentes, **caractérisé en ce que** le ou chaque conduit d'isolation (29A ; 29B) présente une extrémité distale (33A ; 33B) obturée en forme de biseau.

5. Dispositif selon l'une quelconque des revendications précédentes, **caractérisé en ce que** le ou chaque conduit d'isolation (29B) est engagé dans l'ouverture de retenue (23B) et comprend une collerette (87) disposée en appui sur une surface extérieure du tuteur (13), la collerette (87) délimitant le passage distal (29B).

6. Dispositif selon l'une quelconque des revendications précédentes, caractérisé que le ou chaque conduit d'isolation (29A ; 29B) est solidaire du tuteur (13) sur sensiblement toute la longueur du conduit d'isolation (29A ; 29B).

7. Dispositif selon la revendication 6, **caractérisé en ce que** le ou chaque conduit d'isolation (29A ; 29B) est venu de matière avec le tuteur (13).

8. Dispositif selon l'une quelconque des revendications précédentes, caractérisé que le tuteur (13) comprend une ouverture de passage proximale (22B) et une ouverture de passage distale (22A) sensiblement coaxiales, le conduit de guidage dépourvu de lien (41) s'étendant entre ces ouvertures (22A ; 22B) et étant délimité, au voisinage d'au moins une desdites ouvertures de passage (22A ; 22B) par une surface (67) divergeant distalement.

9. Dispositif selon l'une quelconque des revendications précédentes, caractérisé qu'il comprend au moins deux liens filiformes (53A, 53B), les conduits d'isolation (29A, 29B) associés à au moins deux liens (53A, 53B) étant répartis à la périphérie du conduit de guidage (41) dépourvu de lien.

10. Dispositif selon la revendication 9, **caractérisé en ce qu'**il comprend un tuteur unique (13), au moins deux ouvertures de retenue (23A, 23B) décalées longitudinalement étant ménagées dans le tuteur (13).

11. Dispositif selon la revendication 9 ou 10, **caractérisé en ce qu'**il comprend un organe de manoeuvre commun (54) d'au moins deux liens (53A, 53B), l'organe de manoeuvre étant disposé hors du tuteur (13), et **en ce que** les extrémités de commande (77A, 77B) des deux liens (53A, 53B) sont fixées sur l'organe de manoeuvre (54).

12. Dispositif selonl'une quelconque des revendications précédentes, **caractérisé en ce que** chaque conduit d'isolation est fixé au tuteur par son extremité proximale.

13. Dispositif selon une quelconque des revendications précédentes, **caractérisé en ce que** l'implant (11) est une endroprothèse tubulaire, montée de manière coaxiale sur le tuteur (13), le tuteur (13) étant un tuteur unique.

## Claims

1. A device for treating a blood vessel, of the type comprising:
- at least one implant (1) deployable between a contracted state and an expanded state;
- a hollow prop (13) internally delimiting a central passage (14) between a proximal end (21) and a distal end (19), the hollow prop (13), in the vicinity of its distal end (19), defining at least one opening for retaining (23A; 23B) the implant;
- at least one filamentary line (53A; 53B) for releaseably fixing the implant (11) to the prop (13), the or each filamentary line (53A; 53B) comprising a pulling portion (75A; 75B) engaged in the retaining opening (23A; 23B) and extending into the central passage (14) at least as far as a control end (77A; 77B) which can be operated from the proximal end (21) of the prop (13), the device comprising for the or each filamentary line (53A; 53B) an internal insulating duct (29A; 29B) arranged in the central passage (14) and fixed axially with respect to the prop (13), the or each insulating duct (29A; 29B) receiving just one line (53A; 53B) and delimiting, within the central passage (14), a guiding duct (41) with no line (53A; 53B) at least between the proximal end (21) of the prop and a retaining opening (23A; 23B), **characterised in that** the or each insulating duct is formed by a tube which has a cylindrical section.

2. The device according to Claim 1, **characterised in that** the or each insulating duct (29A; 29B) delimits a channel (37A; 37B) for receiving the line extending in cross-section over part of a circumference around a longitudinal axis (X-X') of the central passage (14).

3. The device according to either of Claims 1 or 2, **characterised in that** the or each insulating duct (29A; 29B) has a distal passage (39A; 39B) extending substantially opposite an associated retaining opening (23A, 23B), the or each line (53A; 53B) being engaged in the distal passage (39A; 39B).

4. The device according to any of the preceding claims, **characterised in that** the or each insulating duct (29A; 29B) has a distal end (33A; 33B) which is sealed in a bevelled manner.

5. The device according to any of the preceding claims, **characterised in that** the or each insulating duct (29B) is engaged in the retaining opening (23B) and comprises a collar (87) disposed resting on an external surface of the prop (13), the collar (87) delimiting the distal passage (29B).

6. The device according to any of the preceding claims, **characterised in that** the or each insulating duct (29A; 29B) is integral with the prop (13) over substantially the entire length of the insulating duct (29A; 29B).

7. The device according to Claim 6, **characterised in that** the or each insulating duct (29A; 29B) is made of the same material as the prop (13).

8. The device according to any of the preceding claims, **characterised in that** the prop (13) comprises a proximal passage opening (22B) and a distal passage opening (22A) which are substantially coaxial, the guiding duct with no line (41) extending between these openings (22A; 22B) and being delimited, in the vicinity of at least one of said passage openings (22A; 22B) by a distally diverging surface (67).

9. The device according to any of the preceding claims, **characterised in that** it comprises at least two filamentary lines (53A, 53B), the insulating ducts (29A, 29B) associated with at least two lines (53A, 53B) being arranged around the periphery of the guiding duct (41) with no line.

10. The device according to Claim 9, **characterised in that** it comprises just one prop (13), at least two longitudinally spaced retaining openings (23A, 23B) being provided in the prop (13).

11. The device according to Claim 9 or 10, **characterised in that** it comprises an element for joint movement (54) of at least two lines (53A; 53B), the movement element being disposed outside of the prop (13), and **in that** the control ends (77A; 77B) of the two lines (53A; 53B) are fixed on the movement element (54).

12. The device according to any of the preceding claims, **characterised in that** each insulating duct is fixed to the prop by its proximal end.

13. The device according to any of the preceding claims, **characterised in that** the implant (11) is a tubular endroprosthesis mounted coaxially on the prop (13), the prop (13) being a single prop.

## Patentansprüche

1. Vorrichtung zur Behandlung eines Blutgefäßes des Typs, umfassend:
- mindestens ein Implantat (11), das zwischen einem zusammengezogenen Zustand und einem expandierten Zustand entfaltbar ist;
- einen hohlen Einführkatheter (13), der eine zentrale Durchführung (14) zwischen einem proximalen Ende (21) und einem distalen Ende (19) innen begrenzt, wobei der hohle Einführkatheter (13) in der Nähe seines distalen Endes (19) mindestens eine Halteöffnung (23A, 23B) des Implantats aufweist;
- mindestens ein fadenförmiges Band (53A, 53B) zur lösbaren Befestigung des Implantats (11) am Einführkatheter (13), wobei das bzw. jedes fadenförmige Band (53A, 53B) ein Zugteil (75A, 75B) beinhaltet, das in die Halteöffnung (23A, 23B) eingreift und sich in der zentralen Durchführung (14) mindestens bis zu einem Steuerendstück (77A, 77B) erstreckt, das vom proximalen Ende (21) des Einführkatheters (13) aus betätigt werden kann, wobei die Vorrichtung für das bzw. jedes fadenförmige Band (53A, 53B) ein internes Isolierungsrohr (29A, 29B) in der zentralen Durchführung (14) beinhaltet, das axial am Einführkatheter (13) befestigt ist, und das bzw. jedes Isolierungsrohr (29A, 29B) ein einziges Band (53A, 53B) aufnimmt und in der zentralen Durchführung (14) ein Führungsrohr (41) ohne Band (53A, 53B) zumindest zwischen dem proximalen Ende (21) des Einführkatheters und einer Halteöffnung (23A, 23B) begrenzt, **dadurch gekennzeichnet, dass** das bzw. jedes Isolierungsrohr aus einem Tubus mit zylindrischem Querschnitt besteht.

2. Vorrichtung nach Anspruch 1, **dadurch gekennzeichnet, dass** das bzw. jedes Isolierungsrohr (29A, 29B) einen Aufnahmekanal (37A, 37B) des Bands begrenzt, der sich mit seinem Querschnitt auf einem Teil eines Umfangs um eine Längsachse (X-X') der zentralen Durchführung (14) erstreckt.

3. Vorrichtung nach einem der Ansprüche 1 oder 2, **dadurch gekennzeichnet, dass** das bzw. jedes Isolierungsrohr (29A, 29B) eine distale Durchführung (39A, 39B) aufweist, die sich ungefähr gegenüber einer entsprechenden Halteöffnung (23A, 23B) erstreckt, wobei das bzw. jedes Band (53A, 53B) in der distalen Durchführung (39A, 39B) verläuft.

4. Vorrichtung nach einem der vorangehenden Ansprüche, **dadurch gekennzeichnet, dass** das bzw. jedes Isolierungsrohr (29A, 29B) ein in Form einer Abschrägung verschlossenes distales Ende (33A, 33B) aufweist.

5. Vorrichtung nach einem der vorangehenden Ansprüche, **dadurch gekennzeichnet, dass** das bzw. jedes Isolierungsrohr (29B) in der Halteöffnung (23B) in Eingriff steht und einen Bund (87) beinhaltet, der an einer Außenfläche des Einführkatheters (13) anliegt, wobei der Bund (87) die distale Durchführung (29B) begrenzt.

6. Vorrichtung nach einem der vorangehenden Ansprüche, **dadurch gekennzeichnet, dass** das bzw. jedes Isolierungsrohr (29A, 29B) mit dem Einführkatheter (13) auf etwa der gesamten Länge des Isolierungsrohrs (29A, 29B) verbunden ist.

7. Vorrichtung nach Anspruch 6, **dadurch gekennzeichnet, dass** das bzw. jedes Isolierungsrohr (29A, 29B) aus dem selben Werkstoff wie der Einführkatheter (13) hergestellt wird.

8. Vorrichtung nach einem der vorangehenden Ansprüche, **dadurch gekennzeichnet, dass** der Einführkatheter (13) eine proximale Durchführungsöffnung (22B) und eine annähernd koaxiale distale Durchführungsöffnung (22A) beinhaltet, wobei das Führungsrohr ohne Band (41) sich zwischen diesen Öffnungen (22A, 22B) erstreckt und in der Nähe von mindestens einer der besagten Durchführungsöffnungen (22A, 23B) von einer distal divergierenden Fläche (67) begrenzt wird.

9. Vorrichtung nach einem der vorangehenden Ansprüche, **dadurch gekennzeichnet, dass** sie mindestens zwei fadenförmige Bänder (53A, 53B) besitzt, wobei die mindestens zwei Bändern (53A, 53B) zugeordneten Isolierungsrohre (29A, 29B) am Umfang des Führungsrohrs (41) ohne Band verteilt sind.

10. Vorrichtung nach Anspruch 9, **dadurch gekennzeichnet, dass** sie einen einzigen Einführkatheter (13) besitzt, wobei mindestens zwei in Längsrichtung versetzte Halteöffnungen (23A, 23B) im Einführkatheter (13) angebracht sind.

11. Vorrichtung nach Anspruch 9 oder 10, **dadurch gekennzeichnet, dass** sie ein gemeinsames Betätigungsorgan (54) der mindestens zwei Bänder (53A, 53B) besitzt, wobei das Betätigungsorgan außerhalb des Einführkatheters (13) angeordnet ist, und die Steuerenden (77A, 77B) der beiden Bänder (53A, 53B) am Betätigungsorgan (54) befestigt sind.

12. Vorrichtung nach einem der vorangehenden Ansprüche, **dadurch gekennzeichnet, dass** jedes Isolierungsrohr am Einführkatheter an seinem proximalen Ende befestigt ist.

13. Vorrichtung nach einem der vorangehenden Ansprüche, **dadurch gekennzeichnet, dass** das Implantat (11) eine rohrförmige Endoprothese ist, die koaxial am Einführkatheter (13) angebracht ist, und es sich beim Einführkatheter (13) um einen einzigen Einführkatheter handelt.
